(19) Europäisches Patentamt
European Patent Office
Office européen des brevets

(11) Veröffentlichungsnummer: **0 374 554 B1**

(12) **EUROPÄISCHE PATENTSCHRIFT**

(45) Veröffentlichungstag der Patentschrift: **09.06.93**

(21) Anmeldenummer: **89122270.5**

(22) Anmeldetag: **02.12.89**

(51) Int. Cl.[5]: **C07F 5/02**, C07F 5/06, C07F 7/22, C07F 9/6574, C07F 7/18, C07D 487/08, C07D 493/08, C07D 495/08, //(C07D487/08,277:00,277:00), (C07D493/00,323:00,323:00), (C07D495/00,341:00,341:00)

(54) **Chirale Verbindungen vom beta-Binaphthyltyp, deren Herstellung und deren Verwendung.**

(30) Priorität: **17.12.88 DE 3842554**

(43) Veröffentlichungstag der Anmeldung: **27.06.90 Patentblatt 90/26**

(45) Bekanntmachung des Hinweises auf die Patenterteilung: **09.06.93 Patentblatt 93/23**

(84) Benannte Vertragsstaaten: **CH DE FR GB IT LI**

(56) Entgegenhaltungen: **DE-A- 2 940 336**

(73) Patentinhaber: **BAYER AG**
**W-5090 Leverkusen 1 Bayerwerk(DE)**

(72) Erfinder: **Kaufmann, Dieter, Dr.**
**Peter-Walterscheidt-Strasse 89**
**W-5060 Bergisch Gladbach 2(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 374 554 B1

CHEMICAL ABSTRACTS, Band 108, 4.-25. Januar 1988, Zusammenfassung Nr. 5642u, Columbus, Ohio, USA; M. HE et al.: "Asymmetric reduction of prochiral arylketones with chiral, 2,2'-dihydroxy-1,1'-binaphtyhylborane complexes" & Montsh, Chem. 1987, Nr. 1, Seiten 127-136

JOURNAL OF ORGANOMETALLIC CHEMISTRY, Band 344, 1988, Seiten 277-283; U.-M. GROSS et al.: "Erste asymmetrische Synthese beta-binaphthylgestützter Silane, Stannane und Borane"

TETRAHEDRON LETTERS, Band 29, Nr. 48, 1988, Seiten 6199-6202; M.E. JUNG et al.: "Chrality transfer from silicon to carbon: use of optically pure cyclic silanes with a binaphthalene chiral unit"

JOURNAL OF THE AMERICAN CHEMICAL SOCIETY, Band 95, 18. April 1973, Seiten 2692, 2693; E.B. KYBA et al.: "Chiral Recognition in Molecular Complexing"

## Beschreibung

Es sind schon chirale Verbindungen vom β-Binaphthyltyp bekannt geworden (s. DE-A 29 40 336, C.A. 108 5642u, J. Orgmet. Chem. 344, 277 - 283 (1988), Tetrahedron Letters 29 (48), 6199 - 6202 (1988) und aus J.A.C.S. 95, 2692 - 2693 (1973). Bei diesen Verbindungen ist jeweils eine Binaphthylgruppe an ein Metallatom gebunden oder zwei Binaphthylgruppen über Polyethergruppen miteinander verknüpft. Verbindungen vom β-Binaphthyltyp, bei denen drei Binaphthylgruppen an zwei dreiwertige Zentralatome gebunden sind, sind bisher nicht bekannt geworden.

Die vorliegende Erfindung betrifft neue chirale Verbindungen vom β-Binaphthyltyp der Formel (I)

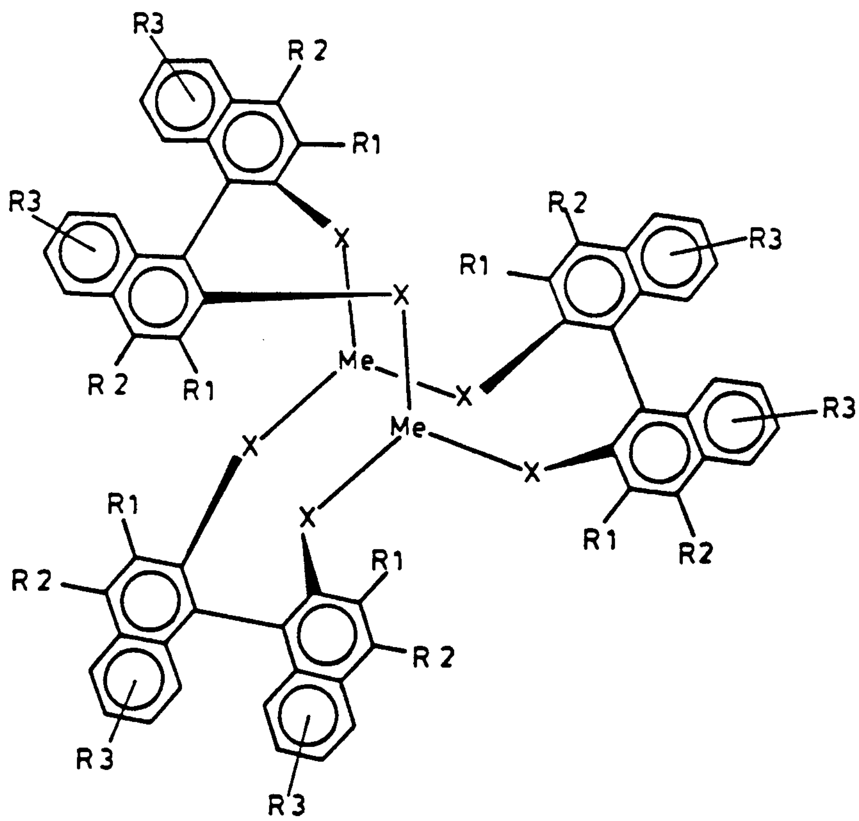

(I),

in der

Me für B, Al, $CR^4$, $SiR^4$, $Sn(IV)R^4$, P oder P=O mit $R^4$ = Wasserstoff, $C_1$- bis $C_6$-Alkyl, Halogen, Phenyl oder O-$C_1$- bis $C_6$-Alkyl,

X für O, S oder $NR^5$ mit $R^5$ = Wasserstoff, $C_1$- bis $C_6$-Alkyl oder Phenyl,

$R^1$ für Wasserstoff, $C_1$- bis $C_6$-Alkyl, Tri-$C_1$- bis $C_4$-alkyl-silyl oder gegebenenfalls substituiertes Phenyl,

$R^2$ und $R^3$ gleich oder verschieden sein können und jeweils für Wasserstoff, $C_1$- bis $C_6$-Alkyl, gegebenenfalls substituiertes Phenyl, Tri-$C_1$- bis $C_4$-alkylsilyl, Halogen oder O-$C_1$- bis $C_6$-Alkyl

stehen.

Soweit $R^2$, $R^3$ und $R^4$ Halogen bedeuten ist Fluor, Chlor und Brom, insbesondere Fluor und Chlor bevorzugt. Soweit $R^1$, $R^2$ und $R^3$ gegebenenfalls substituiertes Phenyl bedeuten kommen als Substituenten beispielsweise $C_1$- bis $C_6$-Alkyl-, Halogen- oder O-$C_1$- bis $C_6$-Alkylreste in Frage.

Bevorzugt sind Verbindungen der Formel (I), bei denen

Me für B, Al, $CR^4$ oder P,

X für O oder S,

$R^1$ für Wasserstoff, $C_1$- bis $C_4$-Alkyl, Tri-$C_1$- bis $C_2$-alkyl-silyl oder Phenyl und

$R^2$ und $R^3$ für Wasserstoff stehen.

Besonders bevorzugt ist die Verbindung der Formel (I) bei der Me für B, X für O und $R^1$ bis $R^3$ für Wasserstoff stehen.

Als weitere beispielhafte Einzelverbindungen der Formel (I) seien die mit X = O, $R^2$ = Wasserstoff und $R^3$ = Wasserstoff und folgenden weiteren Substituenten genannt:

| Me | $R^1$ | $R^4$ |
|---|---|---|
| B | $CH_3$ | - |
| | $C_2H_5$ | |
| | $C_3H_7$ | |
| | $C_6H_5$ | |
| Al | H | - |

| Me | $R^1$ | $R^4$ |
|---|---|---|
| $CR^4$ | H | H |
| | | $CH_3$ |
| | | $C_2H_5$ |
| | | $C_6H_5$ |
| | | F |
| | | Cl |
| | | $OCH_3$ |
| | | $OC_2H_5$ |
| $SiR^4$ | H | H |
| | | $CH_3$ |
| | | $C_2H_5$ |
| | | $C_6H_5$ |
| | | F |
| | | Cl |
| | | $OCH_3$ |
| | | $OC_2H_5$ |
| $SnR^4$ | H | H |
| | | $CH_3$ |
| | | $C_2H_5$ |
| | | $C_6H_5$ |
| | | F |
| | | Cl |
| | | $OCH_3$ |
| | | $OC_2H_5$ |
| P | H | - |
| P = O | H | - |

Die vorliegende Erfindung betrifft weiterhin ein Verfahren zur Herstellung von chiralen Verbindungen vom $\beta$-Binaphthyltyp der Formel (I), das dadurch gekennzeichnet ist, daß man eine $\beta$-Binaphthylverbindung der Formel (II)

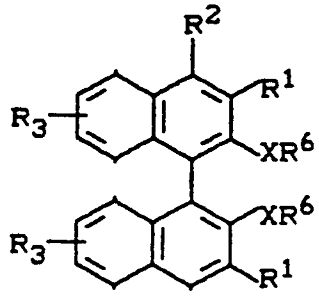

(II),

in der

X, $R^1$, $R^2$ und $R^3$ die bei Formel (I) angegebene Bedeutung haben und

$R^6$ für Wasserstoff, $C_1$- bis $C_6$-Alkyl oder Tri-$C_1$- bis $C_4$-alkyl-silyl steht

mit einer Verbindung der Formel (III) umsetzt

Me $Y^1Y^2Y^3$ (III),

in der

Me die bei Formel (I) angegebene Bedeutung hat und

$Y^1$,$Y^2$ und $Y^3$ gleich oder verschieden sind und jeweils für Wasserstoff, Fluor, Chlor, Brom, Iod, $NH_2$, Mono-$C_1$- bis $C_6$-alkyl-amino, Di-$C_1$-bis $C_6$-alkyl-amino, $C_6$- bis $C_{10}$-Aryl-$C_1$-bis $C_6$-alkyl-amino, Di-$C_6$- bis $C_{10}$-arylamino, OH, $C_1$- bis $C_6$-Alkoxy, $C_6$- bis $C_{10}$-Aryloxy, Tri-$C_1$- bis $C_4$-alkyl-siloxy, $C_1$- bis $C_6$-Thioalkoxy, $C_6$- bis $C_{10}$-Thioaryloxy, $C_1$- bis $C_6$-Alkyl oder $C_6$- bis $C_{10}$-Aryl stehen.

Gegebenenfalls können Verbindungen der Formel (III) in Form von Addukten eingesetzt werden, beispielsweise Borverbindungen als Etherate oder Thioetherate, wie $BF_3$ x $O(C_2H_5)_2$, $BH_3$ x Tetrahydrofuran, $H_2BCl$ x $O(C_2H_5)_2$ oder $H_2BBr$ x $S(CH_3)_2$.

Soweit in den Formeln (II) und (III) identische Symbole wie bei Formel (I) verwendet werden, sind bei den Formeln (II) und (III) auch diejenigen Bedeutungen bevorzugt, die bei Formel (I) als bevorzugt angegeben sind. In Formel (II) steht $R^6$ vorzugsweise für Wasserstoff oder Trimethylsilyl; in Formel (III) stehen $Y^1$, $Y^2$ und $Y^3$ unabhängig voneinander vorzugsweise für Wasserstoff, Chlor, Brom, OH, Methoxy und/oder Ethoxy.

Besonders bevorzugte Verbindungen der Formel (II) sind β-Binaphthol, β-Thiobinaphthol, 2,2′-Diamino-1,1′-binaphthyl und deren Derivate, die in 3- und 3′-Stellung mit Wasserstoff, Methyl, Ethyl, Propyl oder Phenyl substituiert sind.

Besonders bevorzugte Verbindungen der Formel (III) sind Bortrifluorid-diethyletherat, Bortrichlorid, $BH_3$ x Tetrahydrofuran, Chlordihydroboran-diethylether und Bromdihydroboran-dimethylsulfid.

Verbindungen der Formeln (II) und (III) sind entweder bekannt oder sind auf analoge Weise wie die bekannten Verbindungen zugänglich.

In einer besonderen Ausführungsform des erfindungsgemäßen Verfahrens wird in Gegenwart von Säureakzeptoren gearbeitet. Geeignete Säureakzeptoren sind beispielsweise basische Stickstoffverbindungen wie Trialkylamine, Dialkyl-cycloalkylamine, Dialkyl-aralkylamine und Dialkyl-arylamine. Bevorzugt sind Triethylamin, Tripropylamin, Tributylamin, Dimethyl-cyclopentylamin, Dimethyl-cyclohexylamin, Diethyl-cyclopentylamin, Diethyl-cyclohexylamin, Dimethyl-benzylamin, Diethyl-benzylamin und Dimethylanilin.

Falls in Gegenwart von Säureakzeptoren gearbeitet werden soll, können diese beispielsweise in Mengen von 1 bis 10 Mol, bezogen auf 1 Mol der Verbindung der Formel (III) eingesetzt werden.

Das erfindungsgemäße Verfahren kann in Anwesenheit oder Abwesenheit von Verdünnungsmitteln durchgeführt werden. Als Verdünnungsmittel kommen inerte oder weitgehend inerte organische Lösungsmittel in Frage, die unter den jeweils angewendeten Reaktionsbedingungen flüssig sind. Beispiele hierfür sind aliphatische und aromatische Kohlenwasserstoffe, halogenierte Kohlenwasserstoffe, Ether, Ketone, Ester, Nitrile, Amide, Dimethylsulfoxid und Tetramethylensulfon. Im einzelnen seien genannt: Pentan, Hexan, Heptan, Octan, Cyclohexan, Methylcyclohexan, Petrolether, Benzin, Ligroin, Benzol, Toluol, Xylol, Tetralin, Methylenchlorid, Ethylenchlorid, Trichlorethylen, Chloroform, Tetrachlorkohlenstoff, Chlorbenzol, Dichlorbenzol, Diethylether, Dipropylether, Diisopropylether, Dibutylether, Methyl-tert.-butylether, Glykoldimethylether, Diglykoldimethylether, Tetrahydrofuran, Dioxan, Aceton, Methylethylketon, Diethylketon, Methylisopropylketon, Methyl-isobutylketon, Methyl-tert.butylketon, Essigsäuremethylester, Essigsäureethylester, Essigsäurepropylester, Essigsäurebutylester, Essigsäureamylester, Phthalsäuredimethylester, Phthalsäurediethylester,

Acetonitril, Propionitril, Dimethylformamid, Dimethylacetamid und N-Methylpyrrolidon. Besonders bevorzugt sind Methylenchlorid, Chloroform, Chlorbenzol und Dichlorbenzol.

Geeignete Reaktionstemperaturen für das erfindungsgemäße Verfahren sind beispielsweise solche zwischen -100 und +250 °C. Bevorzugt arbeitet man bei -40 bis +150 °C.

Bezogen auf 1 Mol einer Verbindung der Formel (II) kann man beispielsweise 0,6 bis 7 Mol einer Verbindung der Formel (III) einsetzen. Vorzugsweise beträgt dieses Verhältnis 1:0,7 bis 2,0, insbesondere 1:0,8 bis 1,2.

Im allgemeinen arbeitet man bei Normaldruck. Man kann jedoch auch bei erniedrigtem oder erhöhtem Druck arbeiten und beispielsweise Drucke im Bereich von 0,1 bis 10 bar anwenden. Häufig ist ein Arbeiten unter Schutzgas, beispielsweise Stickstoff, vorteilhaft

Bei einer besonderen Ausführungsform des erfindungsgemäßen Verfahrens tropft man eine Lösung der Verbindung der Formel (III) zu einer Lösung der Verbindung der Formel (II) und rührt dann das Reaktionsgemisch nach, z.B. 1 bis 5 Stunden.

Die Aufarbeitung des Reaktionsgemisches ist häufig sehr einfach. Beispielsweise kann man alle flüchtigen Komponenten im Vakuum entfernen und erhält dann eine Verbindung der Formel (I) als festen Rückstand, den man gegebenenfalls noch umkristallisieren kann, z.B. aus Methylenchlorid.

Man kann auf die zuvor beschriebene Weise die Verbindungen der Formel (I) häufig in Ausbeuten von über 90 % der Theorie erhalten.

Mit dem erfindungsgemäßen Verfahren gelingt die Herstellung von Verbindungen der Formel (I) auf einfache Weise, in einem einstufigen Verfahren und in guten bis sehr guten Ausbeuten.

Die vorliegende Erfindung betrifft weiterhin die Verwendung von Verbindungen der Formel (I) zur Herstellung optisch aktiver Verbindungen in angereicherter oder reiner Form. Reaktionen, bei denen mit Hilfe von Verbindungen der Formel (I) als Katalysator optisch aktive Verbindungen in angereicherter oder reiner Form erhalten werden können sind beispielsweise asymmetrische Cycloadditionsreaktionen, wie Diels-Alder-Reaktionen, asymmetrische Photoreaktionen, asymmetrische katalytische Hydrierungen (in diesem Fall dienen Verbindungen der Formel (I) als Liganden für katalytisch wirksame Übergangsmetallkomplexe) und asymmetrische Epoxidierungen. Reaktionen, bei denen mit molaren Mengen von Verbindungen der Formel (I) optisch aktive Verbindungen in angereicherter oder reiner Form erhalten werden können sind beispielsweise asymmetrische Öffnungen von Epoxidringen, asymmetrische Reduktionen von Carbonyl- und Heterocarbonylverbindungen, asymmetrische Hydrometallierungen und asymmetrische Protonierungen.

Von besonderem Vorteil ist dabei, daß man nach diesen katalytischen Reaktionen die eingesetzte Verbindung der Formel (I) oder (II), z.B. durch Hydrolyse, nahezu quantitativ zurückgewinnen kann, ohne daß eine Racemisierung stattfindet.

## Beispiele

### Beispiel 1

Zu einer Lösung von 286,3 mg (1,0 mmol) (S)-(-)-2,2'-Dihydroxy-1,1'-binaphthyl in 25 ml trockenem Dichlormethan wurden unter Stickstoff und bei -20 °C 154,9 mg (1,0 mmol) Monobromboran-dimethylsulfid per Spritze zugetropft. Danach ließ man langsam auf Raumtemperatur erwärmen. Dann wurden alle flüchtigen Bestandteile im Vakuum verdampft und kondensiert. Der verbleibende Rückstand wurde bei 50 °C und 0,1 mbar getrocknet. Es wurden 282,8 mg (= 97 % der Theorie) (-)-Hexanaphtho[2,1-c:1',2'-e:2",1"-j:1"',2"'-1:2"",1""-q:1""',2""'-s]-2,7,9,14,15,20-hexaoxa-1,8-diborabicyclo[6.6.6]eicosa-3,5,10,12,16,18-hexaen, Fp. >350 °C erhalten. -$^1$H-NMR (400 MHz, $CDCl_3$, J in Hz): $\delta$ = 6,64 und 6,68 (AB-System, $^3J$ = 8,8, 4H), 7,06 (d, $^3J$ = 8,3, 2H), 7,22 (ddd, $^3J$ = 8,3, 6,7, $^4J$ = 1,3, 2H), 7,47 (ddd, $^3J$ = 8,1, 6,7, $^4J$ = 1,1, 2H), 7,74 (d, $^3J$ = 8,1, 2H). - Ms (70 eV): m/z 877 (3 %), 876 (22 %), 875 (39 %), 874 (11 %). Röntgenstrukturanalyse: $\rho\bar{1}$, a = 9.3537 (10), b = 14.4558 (13), c = 19.1677 (19) Å, $\alpha$ = 111.931(7), $\beta$ = 91.371(8), $\gamma$ = 100.487(8)°, V = 2352.2 (4) Å$^3$, Z = 2, $d_{ber.}$ = 1.335 gcm$^{-3}$; $Mo_{k\alpha}$-Strahlung (Graphitmonochromator); Scanbereich 3° $\leq 2\theta \leq$ 50°; Gesamtzahl der Reflexe: 8272, beobachtete Reflexe: 5749 ($F_0 \geq 4\sigma$ (F)), R = 0,0526, $R_w$ = 0,0612. Nicolet R3m/V-Röntgenvierkreisdiffraktometer, Daten berechnet mit SHELXTL®-PLUS auf MicroVAX® II.

Mittelwerte wichtiger Bindungslängen (Å) und Winkel (°) im inneren bicyclischen Bereich des Moleküls, die Standardabweichungen der B-O und O-C Bindungen betragen maximal 0,004 Å, die der C-C-Bindungen maximal 0,006 Å

B - O 1,358; O - C 1,392; C - C Verknüpfung der Naphthylgruppen 1,495; C(O) - C (verknüpft) 1,367; C(O) - C (nicht verknüpft) 1.406; C - C (zentrale Bindung der Naphthylgruppen) 1,419; B•••B 3,397; O - B - O 120,0; B - O - C 124,9; O - C - C (verknüpft) 116,8; Torsionswinkel C(O) - C (verknüpft) - C ( verknüpft) -C-

(O) 80

Beispiel 2

282,6 mg (0,32 mmol) der gemäß Beispiel 1 erhaltenen Verbindung wurden in 25 ml trockenem Dichlormethan gelöst und bei -78 °C unter Stickstoff tropfenweise zuerst mit 736 mg (10,5 mmol) Methacrolein und nach 15 Minuten mit 661 mg (10,0 mmol) Cyclopentadien per Spritze versetzt. Es wurde 2 Tage lang bei -78 °C gehalten, dann ließ man das Reaktionsgemisch sich langsam auf 0 °C erwärmen. Dann wurde zweimal mit je 10 ml gesättigter Natriumhydrogencarbonatlösung ausgeschüttelt, alle leicht flüchtigen Bestandteile im Vakuum bei Raumtemperatur abgezogen, dann das Produkt bei 50 °C und 0,01 Torr destilliert. Die Ausbeute betrug 1,16 g (85 % der Theorie) eines Gemisches aus 97,4 Gew.-% (+)-exo- und 2,6 Gew.-% endo-2-Methylbicyclo[2.2.1]hepten-2-carbaldehyd. Der Enantiomerenüberschuß des exo-Produktes wurde $^1$H-NMR-spektroskopisch nach Zugabe von Tris[3-(heptafluorpropylhydroxymethylen)-d-camphorto]europium durch Integration der Aldehydprotonen-Resonanzen zu 90 % bestimmt.

## Patentansprüche

**1.** Chirale Verbindungen von β-Binaphthyltyp der Formel (I)

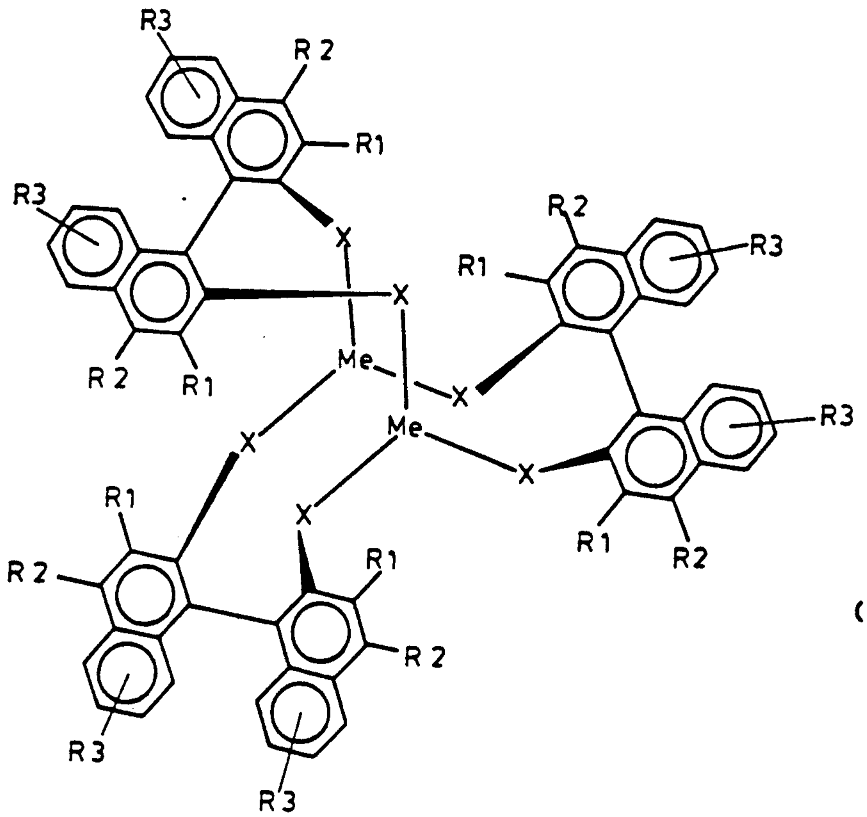

(I)

in der

| | |
|---|---|
| Me | für B, Al, $CR^4$, $SiR^4$, Sn(IV)$R^4$, P oder P=O mit $R^4$ = Wasserstoff, $C_1$- bis $C_6$-Alkyl, Halogen, Phenyl oder O-$C_1$- bis $C_6$-Alkyl, |
| X | für O, S oder $NR^5$ mit $R^5$ = Wasserstoff, $C_1$- bis $C_6$-Alkyl oder Phenyl, |
| $R^1$ | für Wasserstoff, $C_1$- bis $C_6$-Alkyl, Tri-$C_1$- bis $C_4$-alkyl-silyl oder gegebenenfalls substituiertes Phenyl, |
| $R^2$ und $R^3$ | gleich oder verschieden sein können und jeweils für Wasserstoff, $C_1$- bis $C_6$-Alkyl, gegebenenfalls substituiertes Phenyl, Tri-$C_1$- bis $C_4$-alkylsilyl, Halogen oder O-$C_1$- bis $C_6$-Alkyl |

stehen.

**2.** Chirale Verbindungen gemäß Anspruch 1, dadurch gekennzeichnet, daß

| | |
|---|---|
| Me | für B, Al, $CR^4$ oder P, |
| X | für O oder S, |
| $R^1$ | für Wasserstoff, $C_1$- bis $C_4$-Alkyl oder Tri-$C_1$- bis $C_2$-alkyl-silyl oder Phenyl und |
| $R^2$ und $R^3$ | für Wasserstoff stehen. |

**3.** Chirale Verbindung gemäß Ansprüchen 1 und 2, dadurch gekennzeichnet, daß

| | |
|---|---|
| Me | für B, |
| X | für O und |
| $R^1$ bis $R^3$ | für Wasserstoff stehen. |

**4.** Verfahren zur Herstellung von chiralen Verbindungen der Formel (I)

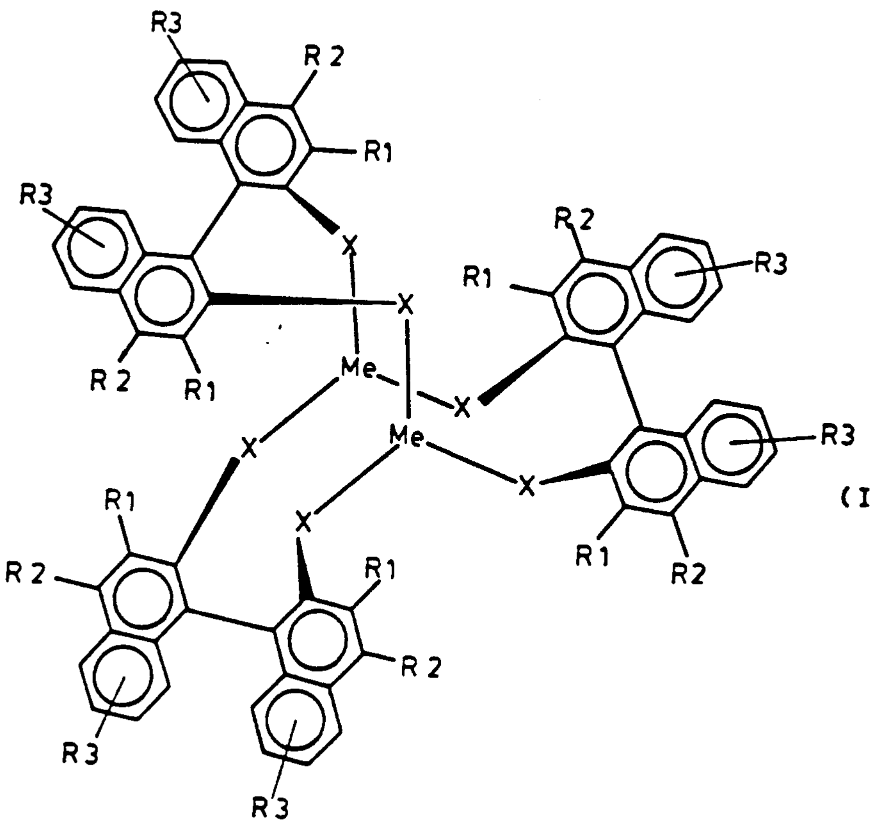

(I),

in der

| | |
|---|---|
| Me | für B, Al, $CR^4$, $SiR^4$, Sn(IV)$R^4$, P oder P=O mit $R^4$ = Wasserstoff, $C_1$- bis $C_6$-Alkyl, Halogen, Phenyl oder O-$C_1$- bis $C_6$-Alkyl, |
| X | für O, S oder $NR^5$ mit $R^5$ = Wasserstoff, $C_1$- bis bis $C_6$-Alkyl oder Phenyl, |
| $R^1$ | für Wasserstoff, $C_1$- bis $C_6$-Alkyl Tri-$C_1$- bis $C_4$-alkyl-silyl oder gegebenenfalls substituiertes Phenyl, |
| $R^2$ und $R^3$ | gleich oder verschieden sein können und jeweils für Wasserstoff, $C_1$- bis $C_6$-Alkyl, gegebenenfalls substituiertes Phenyl, Tri-$C_1$- bis bis $C_4$-alkylsilyl, Halogen oder O-$C_1$- bis $C_6$-Alkyl |

stehen,
dadurch gekennzeichnet, daß man eine $\beta$-Binaphthylverbindung der Formel (II)

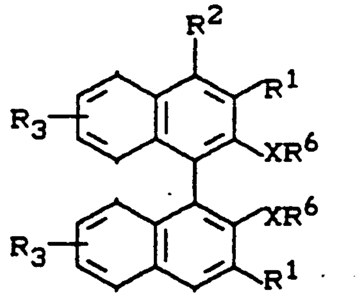

(II),

in der

X, $R^1$, $R^2$ und $R^3$ die bei Formel (I) angegebene Bedeutung haben und

$R^6$ für Wasserstoff, $C_1$- bis $C_6$-Alkyl oder Tri-$C_1$- bis $C_4$-alkyl-silyl steht

mit einer Verbindung der Formel (III) umsetzt

Me $Y^1Y^2Y^3$ (III),

in der

Me die bei Formel (I) angegebene Bedeutung hat und

$Y^1$,$Y^2$ und $Y^3$ gleich oder verschieden sind und jeweils für Wasserstoff, Fluor, Chlor, Brom, Iod, $NH_2$, Mono-$C_1$- bis $C_6$-alkyl-amino, Di-$C_1$-bis $C_6$-alkyl-amino, $C_6$- bis $C_{10}$-Aryl-$C_1$- bis $C_6$-alkyl-amino, Di-$C_6$- bis $C_{10}$-aryl-amino, OH, $C_1$- bis $C_6$-Alkoxy, $C_6$- bis bis $C_{10}$-Aryloxy, Tri-$C_1$- bis $C_4$-alkyl-siloxy, $C_1$- bis $C_6$-Thioalkoxy, $C_6$- bis $C_{10}$-Thioaryloxy, $C_1$- bis $C_6$-Alkyl oder $C_6$- bis $C_{10}$-Aryl stehen,

**5.** Verfahren nach Anspruch 4, dadurch gekennzeichnet, daß man die Verbindungen der Formel (III) in Form von Addukten einsetzt.

**6.** Verfahren nach Ansprüchen 4 und 5, dadurch gekennzeichnet, daß man in Gegenwart von Säureakzeptoren arbeitet.

**7.** Verfahren nach Ansprüchen 4 bis 6, dadurch gekennzeichnet, daß man bei -100 bis +250 °C arbeitet.

**8.** Verfahren nach Ansprüchen 4 bis 7, dadurch gekennzeichnet, daß man bezogen auf 1 Mol einer Verbindung der Formel (II) 0,6 bis 7 Mol einer Verbindung der Formel (III) einsetzt

**9.** Verwendung von Verbindungen der Formel (I) zur Herstellung optisch aktiver Verbindungen in angereicherter oder reiner Form.

## Claims

1. Chiral compounds of the $\beta$-naphthyl type of the formula (I)

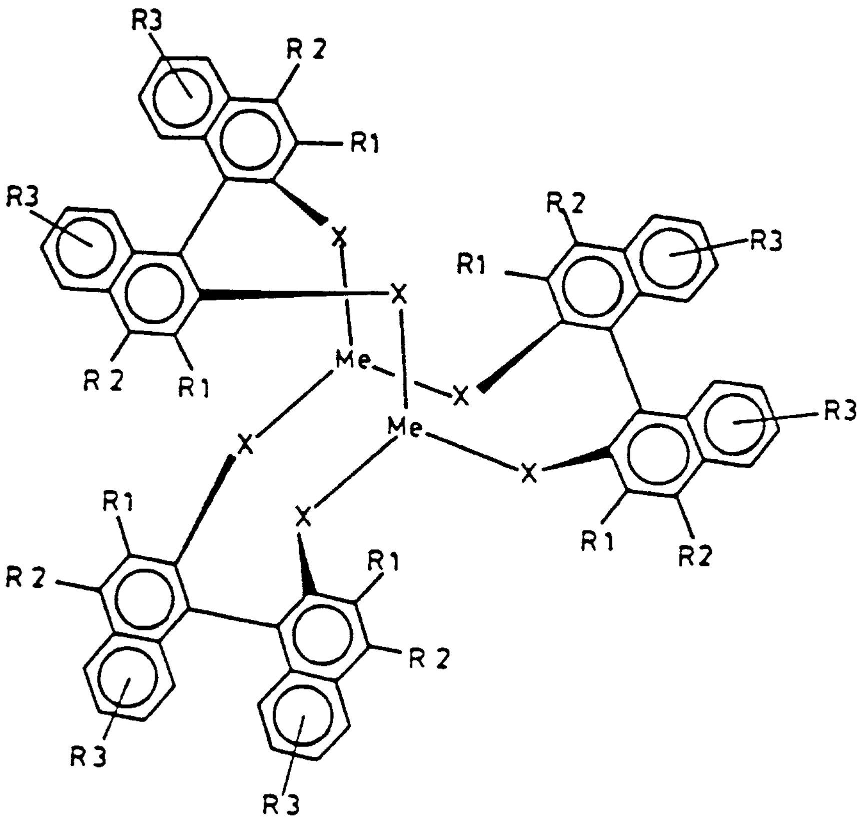

(I)

in which

| | |
|---|---|
| Me | represents B, Al, $CR^4$, $SiR^4$, $Sn(IV)R^4$, P or P=O where $R^4$ is hydrogen, $C_1$-$C_6$-alkyl, halogen, phenyl or O-$C_1$-$C_6$-alkyl, |
| X | represents O, S or $NR^5$ where $R^5$ is hydrogen, $C_1$-$C_6$-alkyl or phenyl, |
| $R^1$ | represents hydrogen, $C_1$-$C_6$-alkyl, tri-$C_1$-$C_4$-alkyl-silyl or substituted or unsubstituted phenyl, |
| $R^2$ and $R^3$ | can be identical or different and each represent hydrogen, $C_1$-$C_6$-alkyl, substituted or unsubstituted phenyl, tri-$C_1$-$C_4$-alkylsilyl, halogen or O-$C_1$-$C_6$-alkyl. |

2. Chiral compounds according to Claim 1, characterized in that

| | |
|---|---|
| Me | represents B, Al, $Cr^4$ or P, |
| X | represents O or S, |
| $R^1$ | represents hydrogen, $C_1$-$C_4$-alkyl or tri-$C_1$-$C_2$-alkyl-silyl or phenyl and |
| $R^2$ and $R^3$ | represent hydrogen. |

3. Chiral compound according to Claims 1 and 2, characterized in that

| | |
|---|---|
| Me | represents B, |
| X | represents O and |
| $R^1$ to $R^3$ | represent hydrogen. |

**4.** Process for the preparation of chiral compounds of the formula (I)

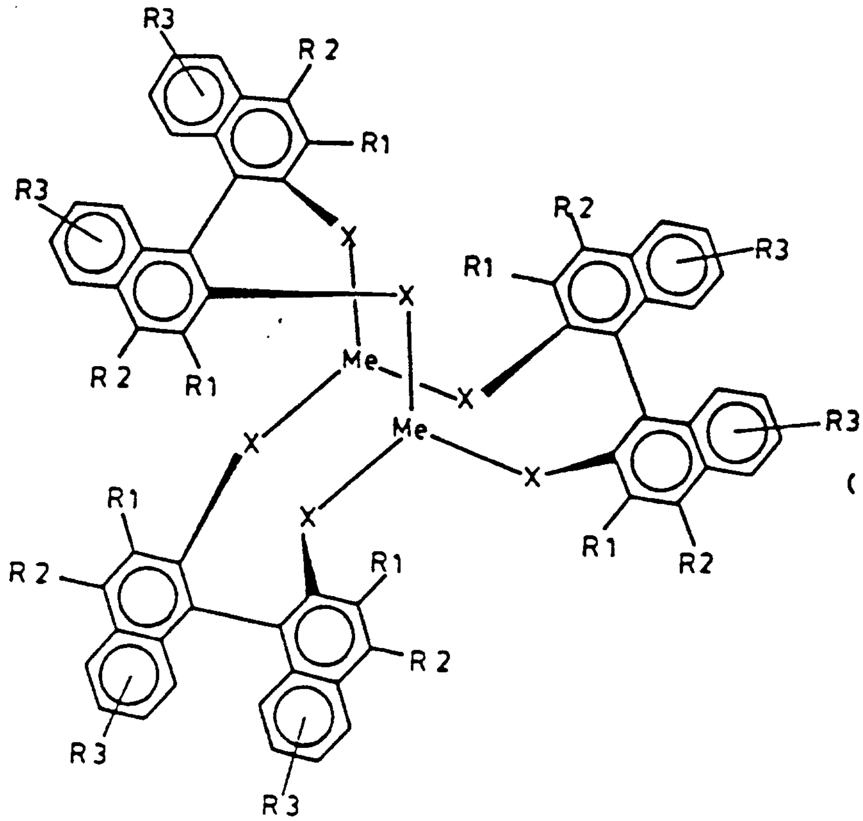

(I),

in which

Me represents B, Al, $CR^4$, $SiR^4$, $Sn(IV)R^4$, P or P=O where $R^4$ is hydrogen, $C_1$-$C_6$-alkyl, halogen, phenyl or O-$C_1$-$C_6$-alkyl,

X represents O, S or $NR^5$ where $R^5$ is hydrogen, $C_1$-$C_6$-alkyl or phenyl,

$R^1$ represents hydrogen, $C_1$-$C_6$-alkyl, tri-$C_1$-$C_4$-alkyl-silyl or substituted or unsubstituted phenyl,

$R^2$ and $R^3$ can be identical or different and each represent hydrogen, $C_1$-$C_6$-alkyl, substituted or unsubstituted phenyl, tri-$C_1$-$C_4$-alkylsilyl, halogen or O-$C_1$-$C_6$-alkyl

which is characterized in that a β-binaphthyl compound of the formula (II)

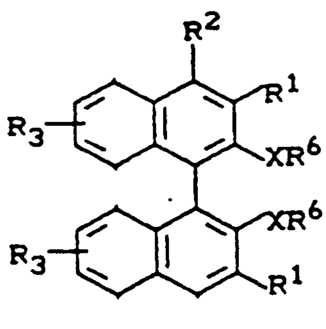

in which

X, $R^1$, $R^2$ and $R^3$ have the meaning mentioned in formula (I) and

$R^6$ represents hydrogen, $C_1$-$C_6$-alkyl or tri-$C_1$-$C_4$-alkyl-silyl

is reacted with a compound of the formula (III)

Me $Y^1Y^2Y^3$ (III),

in which

Me — has the meaning mentioned in formula (I) and

$Y^1$, $Y^2$ and $Y^3$ — are identical or different and each represent hydrogen, fluorine, chlorine, bromine, iodine, $NH_2$, mono-$C_1$-$C_6$-alkyl-amino, di-$C_1$-$C_6$-alkyl-amino, $C_6$-$C_{10}$-aryl-$C_1$-$C_6$-alkyl-amino, di-$C_6$-$C_{10}$-aryl-amino, OH, $C_1$-$C_6$-alkoxy, $C_6$-$C_{10}$-aryloxy, tri-$C_1$-$C_4$-alkylsiloxy, $C_1$-$C_6$-thioalkoxy, $C_6$-$C_{10}$-thioaryloxy, $C_1$-$C_6$-alkyl or $C_6$-$C_{10}$-aryl.

**5.** Process according to Claim 4, characterized in that the compounds of the formula (III) are used in the form of adducts.

**6.** Process according to Claims 4 and 5, characterized in that the process is carried out in the presence of acid acceptors.

**7.** Process according to Claims 4 to 6, characterized in that the process is carried out at -100 to +250 °C.

**8.** Process according to Claims 4 to 7, characterized in that 0.6 to 7 mol of a compound of the formula (III) are used, relative to 1 mol of a compound of the formula (II).

**9.** Use of the compounds of the formula (I) for the preparation of optically active compounds in concentrated or pure form.

## Revendications

**1.** Composés chiraux du type $\beta$-binaphtyle de formule (I)

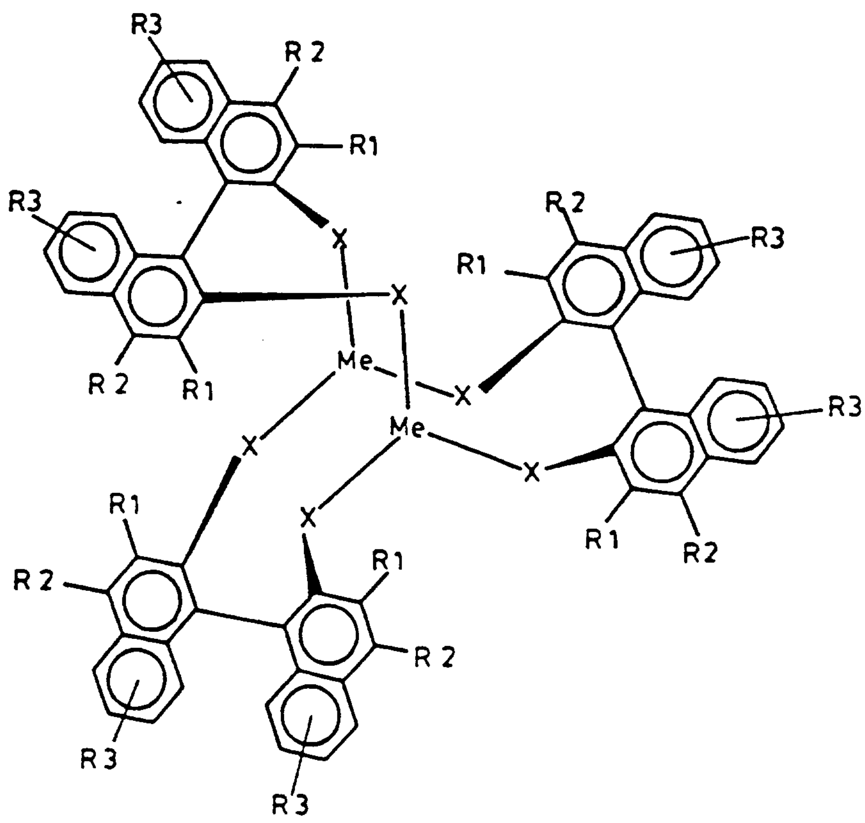

(I)

dans laquelle

Me — représente B, Al, $CR^4$, $SiR^4$, Sn(IV)$R^4$, P ou P=O avec $R^4$ = hydrogène, alkyle en $C_1$ à $C_6$, halogène, phényle ou O(alkyle en $C_1$ à $C_6$),

X — représente O, S ou $NR^5$ avec $R^5$ = hydrogène, alkyle en $C_1$ à $C_6$ ou phényle,

$R^1$ — est l'hydrogène, un groupe alkyle en $C_1$ à $C_6$, tri(alkyle en $C_1$ à $C_4$)silyle ou phényle éventuellement substitué

$R^2$ et $R^3$ — peuvent être identiques ou différents et représentent chacun l'hydrogène, un groupe

alkyle en $C_1$ à $C_6$, phényle éventuellement substitué, tri(alkyle en $C_1$ à $C_4$)silyle, un halogène ou un groupe O-(alkyle en $C_1$ à $C_6$).

2. Composés chiraux suivant la revendication 1, caractérisés en ce que
   - Me représente B, Al, $CR^4$ ou P,
   - X représente O ou S,
   - $R^1$ est l'hydrogène, un groupe alkyle en $C_1$ à $C_4$ ou tri(alkyle en $C_1$ ou $C_2$)silyle ou phényle, et
   - $R^2$ et $R^3$ représentent l'hydrogène.

3. Composé chiral suivant les revendications 1 et 2, caractérisé en ce que
   - Me représente B,
   - X représente O et
   - $R^1$ et $R^3$ représentent l'hydrogène.

4. Procédé de production de composés chiraux de formule (I)

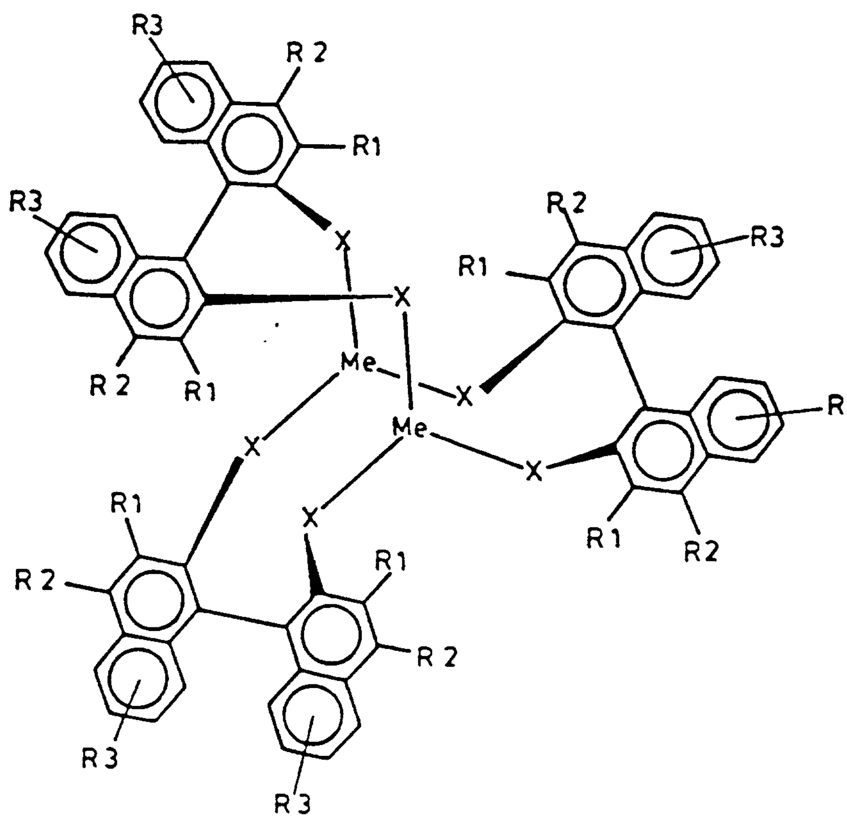

(I)

dans laquelle
   - Me représente B, Al, $CR^4$, $SiR^4$, Sn(IV)$R^4$, P ou P=O avec $R^4$ = hydrogène, alkyle en $C_1$ à $C_6$, halogène, phényle ou O(alkyle en $C_1$ à $C_6$),
   - X représente O, S ou $NR^5$ avec $R^5$ = hydrogène, alkyle en $C_1$ à $C_6$ ou phényle,
   - $R^1$ est l'hydrogène, un groupe alkyle en $C_1$ à $C_6$, tri(alkyle en $C_1$ à $C_4$)silyle ou phényle éventuellement substitué,
   - $R^2$ et $R^3$ peuvent être identiques ou différents et représentent chacun l'hydrogène, un groupe alkyle en $C_1$ à $C_6$, phényle éventuellement substitué, tri(alkyle en $C_1$ à $C_4$)silyle, un halogène ou un groupe O-(alkyle en $C_1$ à $C_6$).

caractérisé en ce qu'on fait réagir un composé $\beta$-binaphtylique de formule (II)

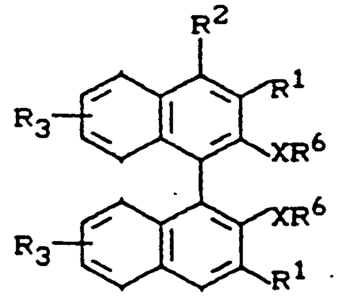

(II)

dans laquelle

X, $R^1$, $R^2$ et $R^3$ ont la définition indiquée pour la formule (I) et

$R^6$ représente l'hydrogène, un groupe alkyle en $C_1$ à $C_6$ ou tri(alkyle en $C_1$ à $C_4$)-silyle

avec un composé de formule (III)

Me $Y^1Y^2Y^3$ (III)

dans laquelle

Me a la définition indiquée pour la formule (I) et

$Y^1$, $Y^2$ et $Y^3$ sont identiques ou différents et représentent chacun l'hydrogène, le fluor, le chlore, le brome, l'iode, un groupe $NH_2$, mono(alkyle en $C_1$ à $C_6$)amino, di(alkyle en $C_1$ à $C_6$)amino, (aryle en $C_6$ à $C_{10}$)-(alkyle en $C_1$ à $C_6$)amino, di(aryle en $C_6$ à $C_{10}$)amino, OH, alkoxy en $C_1$ à $C_6$, aryloxy en $C_6$ à $C_{10}$, tri(alkyle en $C_1$ à $C_4$)siloxy, thioalkoxy en $C_1$ à $C_6$, thioaryloxy en $C_6$ à $C_{10}$, alkyle en $C_1$ à $C_6$ ou aryle en $C_6$ à $C_{10}$.

**5.** Procédé suivant la revendication 4, caractérisé en ce qu'on utilise les composés de formule (III) sous forme de produits d'addition.

**6.** Procédé suivant les revendications 4 et 5, caractérisé en ce qu'on opère en présence d'accepteurs d'acides.

**7.** Procédé suivant les revendications 4 à 6, caractérisé en ce qu'on opère entre -100 et +250 °C.

**8.** Procédé suivant les revendications 4 à 7, caractérisé en ce qu'on utilise, par mole d'un composé de formule (II), 0,6 à 7 moles d'un composé de formule (III).

**9.** Utilisation de composés de formule (I) pour l'obtention de composés optiquement actifs sous une forme concentrée ou pure.